# EUROPEAN PATENT APPLICATION

(11) **EP 1 661 976 A1**
(43) Date of publication of application: **31.05.2006**
(21) Application number: 04027877.2
(22) Date of filing: 24.11.2004
(51) Int. Cl.: C11D 1/06, C11D 3/20, C11D 17/08, C11D 1/83, C11D 10/04, A61K 8/99, A61K 8/18, C11D 1/72

(54) **Ethercarboxylates and glycerine derivatives as foam-enhancing agent for surfactants**

(71) Applicant: KAO CHEMICALS GmbH, 46446 Emmerich (DE)
(72) Inventor: Denzer, Horst, 46446 Emmerich (DE); Meijer, Hamke, 46446 Emmerich (DE); Michaelsen, Marco, 46117 Oberhausen (DE); Abe, Hiroshi, 08210 Barbera del Valles (Barcelona) (ES)
(74) Representative: HOFFMANN EITLE

(57) **Abstract**

The present invention discloses the aqueous compositions comprising
a) an alkyl ether carboxylate having 6 to 22 carbon atoms; and
b) one or more compounds of the following formula (II) : 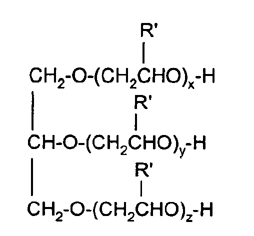 wherein
   R' represents H or CH₃;
   each of x, y and z independently represent a number from 0 to 25, the sum of x, y and z being in the range of 1 to 60, preferably in the range of 2 to 30, more preferably in the range of 3 to 20, even more preferably in the range of 3 to 15,
wherein the weight ratio of component (a) to component (b) is in the range of 4.1:1 to 60:1, and their use as foam-enhancing agent or foam booster for anionic, cationic, non-ionic and/or amphoteric surfactants or mixtures thereof,

## Description

### Technical field

The invention relates to the use of ethercarboxylates and glycerine derivatives as foam-enhancing agent or foam booster for anionic, cationic, non-ionic and/or amphoteric surfactants.

### Prior Art

In a number of surfactant applications, consumers are looking for a high foaming capacity. For example, a shampoo that does not produce enough creamy, stable foam during shampooing has no chance of success on the market. The same applies to manual dishwashing detergents, even though a direct connection between the foaming capacity and the cleaning performance cannot be established at all in many cases.

Hence, besides performance requirements, such as cleaning performance and dermatological compatibility, the foaming behaviour is a further important product feature. However, not all surfactant mixtures which perform satisfactorily and are economical in use show the required foaming behaviour.

The main foaming characteristics of the surfactant formulations that determine their application in areas like personal and house-hold care, washing, food industry, fire fighting, mineral flotation, and many others are: foaming ability, foam stability (foam remaining after certain period at rest), foam quantity (associated with good cleaning effect), creaminess of the foam (associated with conditioning effect), foam density, texture of the foam and foam speed (foam produced after a very short period of time). Insufficient foaming performance can thus for example materialize in an insufficient foaming power or in an insufficient foam stability. In the one case, the basic foam has sufficient height, but it collapses rapidly. In the other case, the exact opposite occurs, i.e. although the initial foaming height is comparably low, the foam remains stable for a long time. Moreover, it is desirable that the foam is obtained quickly upon use (e.g. after a few seconds). Furthermore, the foam must tolerate hard water and the presence of oil.

The number of surfactant combinations that meet this complex requirement profile tends to be small which explains why the same formulations are always found on the market.

One way of overcoming this problem would be to provide surfactant formulations with additives, so-called foam boosters or foam-enhancing agents, which favourably influence the foam properties of the mixtures.

For example, fatty acid alkanolamides can be used as foam boosters, but unfortunately they have the disadvantage that they are suspected of containing traces of nitrosamines which is totally inappropriate for applications where the preparations come into contact with the human skin.

There are several patents and patent applications describing detergent compositions having good foaming properties. For example, EP-A-0702079 discloses a detergent composition comprising component A and component B'
A) a glycerin derivative mixture consisting essentially of 25 to 85 % by weight of a monoester compound of glycerin, 5 to 65 % by weight of di- and triester compounds of glycerin, and 10 to 70 % by weight of an alkylene oxide adduct of glycerin; and
B') at least one surface active agent selected from a monoalkylphosphoric acid, a polyoxyethylene alkyl ether phosphoric acid, a polyoxyethylene alkyl ether acetic acid, an acylated isethionic acid, an N-acylaminoalkylsulfonic acid, a polyoxyethylene alkylsulfosuccinic acid, an N-acylaminoalkylcarboxylic acid, a polyoxyethylene fatty acid alkanolamide ether acetic acid, a fatty acid or an alkyl polyglycoside, or a salt thereof;
   the weight ratio of component (A) to component (B'), (A)/(B), being (5 to 80)/ (95 to 20); and the total concentration of component (A) and component (B') being 5 to 60 by weight, based on the detergent composition.
Said compositions may further comprise a booster (component C) in order to enhance the foaming power. The booster that can be used is selected from an amphoteric surfactant, a fatty acid alkanolamine or an alkylamine oxide.

The German patent application DE-A-19937293 describes the use of alkoxylated carboxylic acid esters of the formula (I): R¹CO(AlkO)ₙOR₂, wherein R₁CO represents an aliphatic acyl radical having 6 to 30 C atoms, AlkO represents an alkylene radical, n represents 1 to 30 and R₂ represents an aliphatic alkylene radical having 1 to 8 carbon atoms. It is indicated that said esters are used as foam booster to improve the foaming properties of anionic, non-ionic, cationic and/or amphoteric or zwitterionic surface-active agents. However, the application does not suggest the combination of an alkoxylated carboxylic acid ester with ethoxylated glycerine and/or ethoxylated and carboxymethylated glycerine.

US-A-5998355 discloses liquid dishwashing detergent compositions that exhibit increased viscosity, better dissolution rate and surprisingly improved cleaning performance in hard water, comprising from about 1% to about 90% of an anionic surfactant and from about 1% to about 30% of a solvent hydrotrope selected from the group consisting of alkoxylated glycerides, alkoxylated glycerines, esters of alkoxylated glycerines, alkoxylated fatty acids, esters of glycerin, polyglycerol esters and combinations thereof. As typical anionic surfactant, carboxylated alcohol ethoxylates are mentioned, among other anionic surfactants. However, it is stated that preferred anionic surfactants include those having at least one sulfur constituent.

The international patent application WO-A-0029530 describes a method for producing low-viscosity aqueous detergent preparations. According to said method, ethylene carboxylic acids or their salts of formula (I) R₁(OCH₂CH₂)ₙOCH₂COOX are added to aqueous solutions of viscosity-reducing additives, R₁ representing a linear and/or branched alkyl-, alkenyl- and/or alkylphenyl radical with 6 to 22 carbon atoms, X representing hydrogen, an alkaline metal, ammonium or alkylammonium and n representing numbers from 1 to 14. Among suitable viscosity-reducing additives, propoxylated glycerine is disclosed. However, WO-A-0029530 fails to address the foaming properties of these low-viscosity aqueous detergent preparations.

Finally, reference is made to EP-A-0580263. This patent relates to the provision of pumpable, highly concentrated (having a solid content of at least 45 wt.-%) liquid aqueous solutions of salts of alkyl ether carboxylic acids derived from alkanols having at least 10 carbon atoms, additionally containing ethoxylated, or ethoxylated and carboxymethylated, products or mixtures thereof, derived from polyhydric alcohols, the degree of ethoxylation of those products being at least 0.5. The ethoxylated, and/or ethoxylated and carboxymethylated products are contained in an amount of at least 1 %, in particular 2 to 25 %, relative to the total amount of solids of ether carboxylic acid and ethoxylated and/or ethoxylated and carboxymethylated products. Again, EP-A-0580263 fails to address the foaming properties of these surfactant mixtures.

### Summary of the invention

Accordingly, the complex problem addressed by the present invention is to provide surface-active preparations which do not show the above-mentioned disadvantages. In particular, the object of the invention is to find foam-enhancing agents that improve different foaming characteristics such as the basic foam, the foam speed, the foam stability, etc., of a large number of surfactants, even in the presence of hard water and oil, which are the most usual conditions for consumers.

The present invention provides aqueous compositions comprising
(a) one or more alkyl ether carboxylates of the following formula I:

   Formula I: R-O-(CH₂CH₂O)ₙ-CH₂COO- M⁺

   wherein
   R is an alkyl residue having 6 to 22 carbon atoms;
   n has a value in the range of 0.3 to 20, more preferably 0.5 to 7, even more preferably 1 to 4.5;
   and M⁺ is an appropriate cation, selected from the group consisting of an alkali metal, an alkaline earth metal, ammonium, an alkylammonium, an alkanolammonium or a glucammonium; and
(b) one or more compounds of the following formula (II): 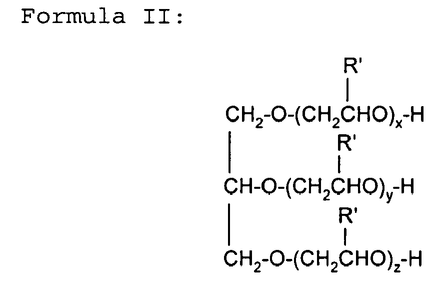
wherein
R' represents H or CH₃, preferably H;
each of x, y and z independently represent a number from 0 to 25, the sum of x, y and z being in the range of 1 to 60, preferably in the range of 2 to 30, more preferably in the range of 3 to 20, even more preferably in the range of 3 to 15, wherein the weight ratio of component (a) to component (b) is in the range of 4.1 to 60:1, preferably 5:1 to 60:1.

### Detailed description of the invention

The aqueous compositions of the present invention comprise
(a) an alkyl ether carboxylate derived from alkanols having 6 to 22 carbon atoms; and
(b) one or more compounds of the following formula (II): 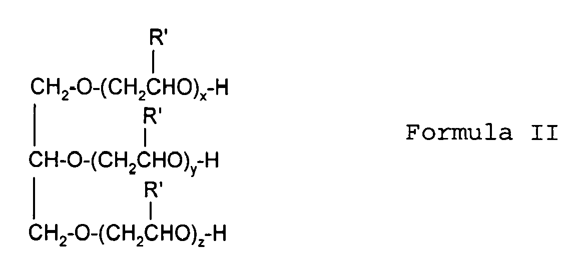 wherein
   R', x, y, and z have the same meaning as defined above,
   the weight ratio of component (a) to component (b) being in the range of 4.1:1 to 60:1, preferably 5:1 to 50:1, more preferably in the range of 6:1 to 40:1, most preferably in the range of 8:1 to 30:1.

The alkyl ether carboxylate derived from alkanols having 6 to 22 carbon atoms used as component (b) is one satisfying the following formula (I):

R-O-(CH₂CH₂O)ₙ-CH₂COO⁻ M⁺ Formula I

wherein R is an alkyl residue having 6 to 22 carbon atoms, n has a value in the range of 0.3 to 20, more preferably 0.4 to 10, even more preferably 0.5 to 7, even more preferably 1 to 4.5, and M+ is an appropriate cation, selected from the group consisting of an alkali metal, an alkaline earth metal, ammonium, an alkylammonium, an alkanolammonium or a glucammonium.

Particularly preferred are compounds of the above formula (I) wherein R is an alkyl residue having 12 to 14 carbon atoms, particularly one having 12 carbon atoms; n has a value in the range of 2 to 4.5 and M⁺ is an alkali metal cation such as sodium or potassium. Particularly preferred is Sodium Laureth-4 Carboxylate.

Alkyl ether carboxylates are obtained by ethoxylation and subsequent carboxymethylation of fatty alcohols (preferably having 6 to 22 carbon atoms).

The process is divided into two steps. The first one is the ethoxylation of alcohols under standard conditions known by the skilled in the art. However, one may also start from commercially available ethoxylated alcohols.

In the second step, the ethoxylated alcohols are reacted with a strong base, like sodium or potassium hydroxide, in presence of a reducing agent, i.e. sodium borohydride, to obtain the corresponding alkoxylate, which is carboxymethylated with sodium monochloroacetate (SMCA).

Optionally, the crude alkyl ether carboxylate acid salt can be purified by first converting the crude products with hydrochloric acid or sulphuric acid into the free acid. Said acid is washed and after that it is converted into the corresponding salt.

Examples of commercially available alkyl ether carboxylate acid salts are marketed under the trade name AKYPO® by Kao Chemicals GmbH.

The compounds used as component (b) of the invention are those satisfying the formula (II): 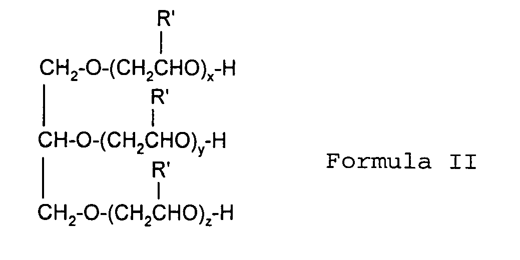 wherein R' represents H or CH₃, preferably H, and each of x, y and z independently represent a number from 0 to 25, the sum of x, y and z being in the range of 1 to 60, preferably in the range of 2 to 30, more preferably in the range of 3 to 15, most preferably in the range of 5 to 10. Particularly preferred are compounds according to formula II
wherein R' is H.

According to the invention, particularly preferred compounds of formula II comprise Glycereth-6, Glycereth-7, Glycereth-9 and Glycereth-12 having an average ethoxylation degree of 6, 7, 9 and 12, respectively.

The composition may also contain (c) ethoxylated fatty alcohols (preferably having 6 to 22 carbon atoms). They are preferably used in a weight ratio of alkyl ether carboxylates (a) / ethoxylated fatty alcohols (c) of 0.5:1 to 1.5:1, more preferably 0.8:1 to 1.2:1, most preferably 1:1. The weight ratio of ethoxylated fatty alcohol (c) / ethoxylated glycerine (b) is preferably in the same range as the weight ratio of alkyl ether carboxylate (b) to ethoxylated glycerine (b) .

The foam booster of the present invention generally does not contain ethoxylated and carboxymethylated glycerine derivatives.

The foam booster of the present invention may be used to improve the foam properties anionic, cationic, non-ionic and/or amphoteric surfactants or mixtures thereof in the form of aqueous solution. Those aqueous solutions which are used to enhance the foaming of other surfactant composition (in the following referred to as "foam booster") preferably contain 15 to 75 wt.%, more preferably 18 to 63 wt.%, of components (a), i.e. the ethercarboxylate, and (b), i.e. the glycerine derivative, as defined above. The amount of component (a) is preferably in the range of 10 to 45 wt.%, more preferably 14 to 30 wt.%. The amount of component (b) is preferably in the range of 1 to 30 wt.%, more preferably 2 to 15 wt.%. The active matter content of the foam booster is also preferably within the range 15 to 80 wt.%, more preferably 18 to 75 wt.%, even more preferably 25 to 62 wt.%. The foam booster of the present invention is generally flowable at room temperature. The active matter content is substantially provided by components (a) and (b).

While the foam booster thus does not include significant amounts of other surfactants, it may include ethoxylated alcohol (component (c)), sodium chloride and impurities, as a result of the preparation process of the alkyl ether carboxylates. The foam booster of the present invention generally does not contain a peaked (narrow range) short chain alcohols since this would unnecessarily increase the costs.

### Anionic surfactants

Typical examples of anionic surfactants are soaps, preferably C12-C18 fatty acid soaps, like soaps derived from lauric acid, myristic acid, palmitic acid, stearic acid, isostearic acid, oleic acid or linoleic acid. Said soaps contain an appropriate cation, selected from the group consisting of an alkali metal, an alkaline earth metal, ammonium, an alkylammonium, an alkanolammonium or a glucammonium. Preferably the cation group is selected from the group consisting of sodium, potassium and triethanolamine. Preferred C12-C18 fatty acid soaps include triethanolamine stearate, triethanolamine palmitate, triethanolamine myristate, triethanolamine laurate, sodium stearate, sodium palmitate, sodium myristate, sodium laurate, potassium stearate, potassium palmitate, potassium myristate, and potassium laurate.

Other examples of anionic surfactants are alkylbenzenesulfonates, alkanesulfonates, olefin sulfonates, alkylether sulfonates, glycerol ether sulfonates, α-methyl ester sulfonates, sulfofatty acids, alkylsulfates, fatty alcohol ether sulfates, glycerol ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, amide ether carboxylic acids and salts thereof, fatty acid isethionates, fatty acid sarcosinates, fatty acid taurides, N-acylamino acids such as, for example, acyl lactylates, acyl tartrates, acyl glutamates, acyl aspartates, alkyl oligoglucoside sulfates, protein fatty acid condensates (particularly wheat-based vegetable products) and alkyl (ether) phosphates.

One of the preferred anionic surfactant is sodium lauryl ether sulfate, particularly preferred is sodium lauryl ether sulfate having an average degree of ethoxylation of 1 to 3, preferably 1 to 2.5, most preferably 2 to 2.5.

### Cationic surfactants

Typical examples of cationic surfactants are amine salts, quaternary ammonium salts (quats) like monoalkyl dimethyl amine derivatives, dialkyl monomethyl amines and imidazoline derivatives, and the quaternized derivatives of polyalkanolamine esters (esterquats). Examples of commercially available quats are: Quartamin® AB (Behentrimonium Chloride), Quartamin® 60W25 (Cetrimonium Chloride) and Quartamin® ABK (Behentrimonium Chloride and Cetearyl Alcohol), all marketed by KAO Corporation S.A.

Examples of commercially available esterquats are Quartamin® BTC-131 (Behenoyl PG-Trimonium Chloride), marketed by KAO Chemicals GmbH, and Tetranyl® CO-40 (Dioleoylethyl Hydroxyethylmonium Methosulfate and Dipropylene Glycol) marketed by KAO Corporation S.A.

### Non-ionic surfactants

Specific examples of non-ionic surfactants are alkoxylated trimethyolol propane, alkoxylated 1,2,3-trihydroxy hexane, alkoxylated pentaetrythritol, alkoxylated sorbitol, alkoxylated glycerol fatty acid ester, alkoxylated trimethyolol propane fatty acid ester, alkoxylated 1,2,3-trihydroxy hexane fatty acid ester, alkoxylated pentaetrythritol fatty acid ester, alkoxylated sorbitol fatty acid ester, fatty alcohol polyglycol ethers, alkylphenol polyglycol ethers, fatty acid polyglycol esters, fatty acid amide polyglycol ethers, fatty amine polyglycol ethers, mixed ethers and mixed formals, optionally partly oxidized alk(en)yl oligoglycosides or glucuronic acid derivatives, fatty acid-N-alkylglucamides, protein hydrolyzates (particularly wheat-based vegetable products), polyol fatty acid esters, sugar esters, alkyl polyglucosides, sorbitan esters, polysorbates. and alkanolamides, including alkoxylated alkanolamides, preferably ethoxylated alkanolamides derived from rapeseed oil, and alkyl ether carboxylic acid alkanolamides, preferably ethoxylated alkyl C11-C13 carboxylic acid alkanolamides.

### Amphoteric surfactants

Specific examples of amphoteric surfactant are alkyl amine oxides, alkyl betaines, alkyl sulphobetaines (sultaines), amidoalkyl betaines, alkyl glycinates, alkyl carboxyglycinates, alkyl amphoacetates, alkyl amphopropionates, alkylamphoglycinates, alkyl amidopropyl betaines, alkyl amidopropyl- and hydroxysultaines. Particularly preferred amphoteric surfactants are alkyl amine oxides, alkyl sulphobetaines (sultaines), alkylamphoglycinates alkyl amphoacetates such as sodium coco monoamphoacetate or sodium coco diamphoacetate, and alkyl amidopropyl betaines such as cocoamido propyl betaine.

### The cosmetic composition

The foam booster of the present invention is used for the production of personal hygiene and/or personal care cosmetic compositions in which they may be present in quantities of 0.5 to 20, preferably 1 to 15 even more preferably 1 to 10, more particularly 1 to 7% by weight, based on the cosmetic compositions.

The cosmetic compositions are, for example liquid soaps, hair shampoos, body shampoos, hair lotions, foam baths, shower baths, creams, gels, lotions, shaving preparations, after-shaving preparations, alcoholic and aqueous/alcoholic solutions or emulsions.

The cosmetic composition is for example obtainable by blending
(A) one or more surfactants selected from an anionic surfactant, a cationic surfactant, an amphoteric surfactant and/or a non-ionic surfactant, e.g. one or more of the surfactant indicated above, and
(B) the foam booster of the present invention,
   wherein the weight ratio (A)/(B) is between 1:1 and 8:1.

The cosmetic composition preferably contains component (A) in an amount of at least 50 wt.% of an anionic surfactant. Most preferably, the anionic surfactant is mainly composed of sodium lauryl ether sulfate or a C12-C18 fatty acid soap. The surfactant (A) does not comprise compounds included in the composition (B), i.e. ethoxylated, non-carboxylated glycerines, alkyl ether carboxylate, and/or ethoxylated fatty alcohols.

The cosmetic composition of the present invention is preferably a liquid soap, hair shampoo, body shampoo, hair lotion, foam bath, shower bath, cream, gel, lotion, shaving preparation, after-shaving preparation, alcoholic and aqueous/alcoholic solution, or emulsion.

In the cosmetic compositions of the present invention, the total amount of surfactants (active matter) (A) is preferably in the range of 10 to 35 wt.-% with respect to the total weight of the composition and the total amount of component (B) is preferably in the range of 0.5 to 20 wt.-%, preferably 1 to 15 wt.-%, most preferred 2 to 10 wt.-%, with respect to the total weight of the composition.

If the cosmetic compositions are hair shampoos or body shampoos the total amount of wash active matter, that is, the total amounts of surfactants contained in the composition of the present invention is preferably less than 30 wt.-%. That is, if the composition contains surfactants other than components (a) and (b), and the total amount of these surfactants and components (a) and (b) and does desirably not exceed 30 wt.-%.

Particularly preferred cosmetic compositions are liquid soaps comprising
a) an alkyl ether carboxylate
b) alkoxylated glycerine
c) one or more C12-C18 fatty acid soaps
d) water.

Preferably, the C12-C18 fatty acid soaps are selected from the group consisting of triethanolamine stearate, triethanolamine palmitate, triethanolamine myristate, triethanolamine laurate, sodium stearate, sodium palmitate, sodium myristate, sodium laurate, potassium stearate, potassium palmitate, potassium myristate, and potassium laurate or mixtures thereof.

It is further preferred that said liquid soaps additionally contain (e) an ethoxylated rapeseed fatty acid amide. A preferred ethoxylated rapeseed fatty acid amide according to the invention is PEG-4 rapeseed amide.

The preferred liquid soaps according to the invention comprise
a) 0.5 to 15 wt.% of an alkyl ether carboxylate as described before
b) 0.5 to 10 wt.% of alkoxylated glycerine as described before
c) 3 to 15 wt.% of one or more C12-C18 fatty acid soaps as described before
d) 53 to 98 wt.% of water
e) 1 to 7 wt.% of an ethoxylated rapeseed fatty acid amide.

The pH value of the liquid soaps according to the invention is preferably in the range of 7 to 11.

### Auxiliaries and additives

In one preferred embodiment of the invention, the preparations may contain other anionic, nonionic, cationic, amphoteric and/or zwitterionic co-emulsifiers as an optional component (d), their percentage content-based on the preparation--being from 0.5 to 10, preferably from 1 to 8 and more particularly from 2 to 5% by weight. Suitable co-emulsifiers are, for example, nonionic surfactants from at least one of the following groups:
(1) products of the addition of 2 to 30 moles of ethylene oxide and/or 0 to 5 moles of propylene oxide onto linear fatty alcohols containing 8 to 22 carbon atoms, onto fatty acids containing 12 to 22 carbon atoms and onto alkylphenols containing 8 to 15 carbon atoms in the alkyl group;
(2) ethoxylated glycerides as described in the European patent applications EP-A-0586323 and EP-A-1045021, preferably obtained by reaction of triglycerides, glycerine and ethylene oxide;
(3) glycerol monoesters and diesters and sorbitan monoesters and diesters of saturated and unsaturated fatty acids containing 6 to 22 carbon atoms and ethylene oxide adducts thereof;
(4) alkyl mono- and oligoglycosides containing 8 to 22 carbon atoms in the alkyl group and ethoxylated analogs thereof;
(5) products of the addition of 15 to 60 moles of ethylene oxide onto castor oil and/or hydrogenated castor oil;
(6) polyol esters and, in particular, polyglycerol esters such as, for example, polyglycerol polyricinoleate, polyglycerol poly-12-hydroxy-stearate or polyglycerol dimerate isostearate. Mixtures of compounds from several of these classes are also suitable;
(7) products of the addition of 2 to 15 moles of ethylene oxide onto castor oil and/or hydrogenated castor oil;
(8) partial esters based on linear, branched, unsaturated or saturated C6-22 fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
(9) mono-, di- and trialkyl phosphates and mono-, di- and/or tri-PEG-alkyl phosphates and salts thereof;
(10) wool wax alcohols;
(11) polysiloxane/polyalkyl polyether copolymers and corresponding derivatives;
(12) mixed esters of pentaerythritol, fatty acids, citric acid and fatty alcohol and/or mixed esters of fatty acids containing 6 to 22 carbon atoms, methyl glucose and polyols, preferably glycerol or polyglycerol,
(13) polyalkylene glycols and
(14) glycerol carbonate.

The addition products of ethylene oxide and/or propylene oxide with fatty alcohols, fatty acids, alkylphenols, glycerol mono- and diesters and sorbitan mono- and diesters of fatty acids or with castor oil are known commercially available products. They are homolog mixtures of which the average degree of alkoxylation corresponds to the ratio between the quantities of ethylene oxide and/or propylene oxide and substrate with which the addition reaction is carried out.

Ethoxylated glycerides according to the invention are preferably a mixture of compounds of the following formula (III) : 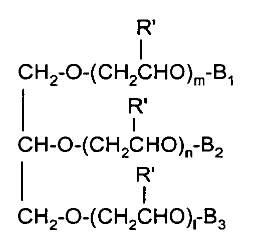 wherein
R' represents H or CH₃;
each m, n, and 1 independently represent a number from 0 to 40, the sum of m, n and 1 being in the range of 1 to 100, preferably 1 to 20, and said mixtures comprising
(i) compounds represented by formula (I), where one of B1, B2 and B3 represents an acyl group having 6 to 22 carbon atoms, the remainder representing H;
(ii) compounds represented by formula (I), where two of B1, B2 and B3, independently, represent an acyl group having 6 to 22 carbon atoms, the remainder representing H;
(iii) compounds represented by formula (I), where each B1, B2 and B3, independently, represent an acyl group having 6 to 22 carbon atoms;
(iv) compounds represented by formula (I), where each of B1, B2 and B3 represent H;
the weight ratio of the compounds (i) / (ii) /(iii) being 46-90/9-35/1-15. Particularly preferred are compounds of the formula III wherein the weight ratio (i)+(ii)+(iii)/(iv) is in the range of 85/15 to 40/60, more preferably in the range 80/20 to 45/55.

C8-18 alkyl mono- and oligoglycosides, their production and their use as surfactants are known from the art. They are produced in particular by reaction of glucose or oligosaccharides with primary alcohols containing 8 to 18 C atoms. So far as the glycoside component is concerned, both monoglycosides, in which a cyclic sugar unit is attached to the fatty alcohol by a glycoside linkage, and oligomeric glycosides with a degree of oligomerization of preferably up to about 8 are suitable. The degree of oligomerization is a statistical mean value on which a homolog distribution typical of such technical products is based.

Zwitterionic surfactants may also be used as emulsifiers. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known by the CTFA name of Cocamidopropyl Betaine is particularly preferred. Other suitable emulsifiers are ampholytic surfactants. Ampholytic surfactants are surface-active compounds which, in addition to a C8-18 alkyl or acyl group, contain at least one free amino group and at least one -COOH or -SO₃H group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C12-18 acyl sarcosine. Suitable anionic emulsifiers are, in particular, alkyl (ether) sulfates, acyl glutamates, protein fatty acid condensates and monoglyceride sulfates. Besides ampholytic emulsifiers, quaternary emulsifiers may also be used, those of the esterquat type, preferably methyl-quaternized difatty acid triethanolamine ester salts, being particularly preferred.

Suitable oil components are, for example, Guerbet alcohols based on fatty alcohols containing 6 to 18 and preferably 8 to 10 carbon atoms, esters of linear C6-22 fatty acids with linear C6-22 fatty alcohols, esters of branched C6-13 carboxylic acids with linear C6-22 fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6-22 fatty acids with branched alcohols, more particularly 2-ethyl hexanol, esters of hydroxycarboxylic acids with linear or branched C6-22 fatty alcohols, more especially Dioctyl Malate, esters of linear and/or branched fatty acids with polyhydric alcohols (for example propylene glycol, dimer diol or trimer triol) and/or Guerbet alcohols, triglycerides based on C6-10 fatty acids, liquid mono-/di-/triglyceride mixtures based on C6-18 fatty acids, esters of C6-22 fatty alcohols and/or Guerbet alcohols with aromatic carboxylic acids, more particularly benzoic acid, esters of C2-12 dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C6-22 fatty alcohol carbonates, Guerbet carbonates, esters of benzoic acid with linear and/or branched C6-22 alcohols, linear or branched, symmetrical or nonsymmetrical dialkyl ethers containing 6 to 22 carbon atoms per alkyl group, ring opening products of epoxidized fatty acid esters with polyols, silicone oils and/or aliphatic or naphthenic hydrocarbons, for example squalane, squalene or dialkyl cyclohexanes.

Suitable secondary consistency factors are hydroxyfatty alcohols, partial glycerides, fatty acids or hydroxyfatty acids. Suitable thickeners are, for example, Aerosil types (hydrophilic silicas), polysaccharides, more particularly xanthan gum, guar guar, agar agar, alginates and tyloses, carboxymethyl cellulose and hydroxyethyl cellulose, relatively high molecular weight polyethylene glycol monoesters and diesters of fatty acids, polyacrylates (for example Carbopols [Goodrich] or Synthalens [Sigma]), polyacrylamides, polyvinyl alcohol and polyvinyl pyrrolidone, surfactants such as, for example, ethoxylated fatty acid glycerides, esters of fatty acids with polyols such as, for example, pentaerythritol or trimethylol propane, narrow-range fatty alcohol ethoxylates or alkyl oligoglucosides and electrolytes, such as sodium chloride and ammonium chloride.

Suitable cationic polymers are, for example, cationic cellulose derivatives such as, for example, the quaternized hydroxyethyl cellulose obtainable from Amerchol under the name of Polymer JR 400, cationic starch, copolymers of diallyl ammonium salts and acrylamides, quaternized vinyl pyrrolidone/vinyl imidazole polymers such as, for example, Luviquat (BASF), condensation products of polyglycols and amines, quaternized collagen polypeptides such as, for example, Lauryidimonium Hydroxypropyl Hydrolyzed Collagen (Lamequat L, Grunau), quaternized wheat polypeptides, polyethyleneimine, cationic silicone polymers such as, for example, Amodimethicone, copolymers of adipic acid and dimethylamino-hydroxypropyl diethylenetriamine (Cartaretine , Sandoz), copolymers of acrylic acid with dimethyl diallyl ammonium chloride (Merquat 550, Chemviron), polyaminopolyamides and crosslinked water-soluble polymers thereof, cationic chitin derivatives such as, for example, quaternized chitosan, optionally in micro-crystalline distribution, condensation products of dihaloalkyls, for example dibromobutane, with bis-dialkylamines, for example bis-dimethylamino-1,3-propane, cationic guar gum such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Preferred silicone compounds are hydrophobic silicone oils, which are silicone oils which are soluble in paraffinic oil at 25°C. Hydrophobic silicone oils to be used according to the present invention include both volatile and non-volatile silicone oils.

Specific examples include a cyclic methyl siloxane having the formula {(CH3)2SiO}x in which x is 3-6, or short chain linear methyl siloxanes having the formula ((CH3)2SiO{(CH3)2SiO}ySi(CH3)3 in which y is 0-5.

Some suitable cyclic methyl siloxanes are hexamethylcyclotrisiloxanes (D3), a solid with a boiling point of 134°C and the formula {(Me2)SiO}3; octamethylcyclotetrasiloxane (D4) with a boiling point of 176°C, a viscosity of 2.3 mm2/s, and the formula {(Me2)SiO}4; decamethylcyclopentasiloxane (D5) (cyclomethicone) with a boiling point of 210°C, a viscosity of 3.87 mm2/s, and the formula {(Me2)SiO}5; and dodecamethylcyclohexasiloxane (D6) with a boiling point of 245°C, a viscosity of 6.62 mm2/s and the formula {(Me2)SiO}6.

Some suitable short linear methyl siloxane are hexamethyldisiloxane (MM) with a boiling point of 100°C, viscosity of 0-65 mm2/s, and formula Me3SiOMe3; octamethyltrisiloxane (MDM) with a boiling point of 152°C, viscosity of 1.04 mm2/s, and formula Me3SiOMe2SiOsiMe3; decamethyltetrasiloxane (MD2M) with a boiling point of 194°C, viscosity of 1.53 mm2/s, and formula Me3SiO(MeSiO)2SiMe3; dodecamethylpentasiloxane (MD3M) with a boiling point of 229°C, viscosity of 2.06 mm2/s, and formula Me3SiO(Me2SiO)3SiMe3; tetradecamethylhexasiloxane (MD4M) with a boiling point of 245°C, viscosity of 2.63 mm2/s, and formula Me3SiO(Me2SiO)4SiMe3; and hexadecamethylheptasiloxane (MD5M) with a boiling point of 270°C, viscosity of 3.24 mm2/s, and formula Me3SiO(Me2SiO)5SiMe3.

Furthermore, long chain linear siloxanes such as phenyltrimethicone, bis(phenylpropyl)dimethicone, dimethicone, and dimethiconol are also included.

Typical examples of fats are glycerides while suitable waxes are inter alia natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

In the context of the invention, biogenic agents are, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Suitable deodorizers are, for example, antiperspirants, such as aluminium chlorhydrates. These antiperspirants are colorless hygroscopic crystals which readily deliquesce in air and which accumulate when aqueous aluminium chloride solutions are concentrated by evaporation. Besides the chlorhydrates, aluminium hydroxylactates and acidic aluminium/zirconium salts may also be used. Other suitable deodorizers are esterase inhibitors, preferably trialkyl citrates, such as trimethyl citrate, tripropyl citrate, triisopropyl citrate, tributyl citrate and, in particular, triethyl citrate. Esterase inhibitors inhibit enzyme activity and thus reduce odor formation. The free acid is probably released through the cleavage of the citric acid ester, reducing the pH value of the skin to such an extent that the enzymes are inhibited. Other esterase inhibitors are sterol sulfates or phosphates, for example lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, for example citric acid, malic acid, tartaric acid or tartaric acid diethyl ester. Antibacterial agents which influence the germ flora and destroy or inhibit the growth of perspiration-decomposing bacteria, may also be present in stick products. Examples of such antibacterial agents are chitosan, phenoxyethanol and chlorhexidine gluconate. 5-Chloro-2-(2,4-dichlorophenoxy)-phenol.

Suitable antidandruff agents are climbazol, octopirox and zinc pyrithione. Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds. Suitable swelling agents for aqueous phases are montmorillonites, clay minerals, Pemulen and alkyl-modified Carbopol types (Goodrich).

Examples of UV protection factors include organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)-benzolc acid-2-ethylhexyl ester, 4-(dimethylamino)-benzoic acid-2-octyl ester and 4-(dimethylamino)-benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzo-phenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1, 3,5-triazine and Octyl Triazone;
- propane-1,3-diones such as, for example, 1-(4-tert.butylphenyl)-3-(4'-methoxyphenyl)-propane-1, 3-dione;
- 2-phenylbenzimidazole-5-sulfonic acid and alkali metal, alkaline earth metal, ammonium, alkylammonium, alkanolammonium and glucam-monium salts thereof;
- sulfonic acid derivatives of benzophenones, preferably 2-hydroxy-4-methoxybenzophenone-5-sulfonic acid and salts thereof;
- sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example 1-(4'-tert.butylphenyl)-3-(4'-methoxyphenyl)-propane-1,3-dione, 4-tert-butyl-4'-methoxydibenzoylmethane (Parsol 1789) or 1-phenyl-3-(4'-isopropylphenyl)-propane-1,3-dione.

The UV-A and UV-B filters may of course also be used in the form of mixtures. Besides the soluble substances mentioned, insoluble pigments, i.e. finely dispersed metal oxides or salts, may also be used for this purpose. Examples of suitable metal oxides are, in particular, zinc oxide and titanium dioxide and also oxides of iron, zirconium, silicon, manganese, aluminium and cerium and mixtures thereof. Silicates (talcum), barium sulfate and zinc stearate may be used as salts. The oxides and salts are used in the form of the pigments for skin-care and skin-protecting emulsions and decorative cosmetics. The particles should have an average diameter of less than 100 nm, preferably from 5 to 50 nm and more preferably from 15 to 30 nm. They may be spherical in shape although ellipsoidal particles or other non-spherical particles may also be used. The pigments may also be surface-treated, i.e. hydrophilicized or hydrophobicized. Typical examples are coated titanium dioxides such as, for example, Titandioxid T 805 (Degussa) or Eusolex T2000 (Merck). Suitable hydrophobic coating materials are, above all, silicones and especially trialkoxyoctyl silanes or simethicones. So-called micro- or nanopigments are preferably used in sun protection products. Micronized zinc oxide is preferably used.

Besides the two above-mentioned groups of primary protection factors, secondary protection factors of the antioxidant type may also be used. Secondary sun protection factors of the antioxidant type interrupt the photochemical reaction chain which is initiated when UV rays penetrate into the skin. Typical examples of suitable antioxidants are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages (also (metal) chelators (for example (α-hydroxyfatty acids, palmitic acid, phytic acid, lactoferrine), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example γ-linolenic acid, linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, (α-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxy-butyrophenone, uric acid and derivatives thereof, mannose and derivatives thereof, Superoxid-Dismutase, zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

In addition, hydrotropes such as, for example, ethanol, isopropyl alcohol or polyols may be used to improve flow behavior. Suitable polyols preferably contain 2 to 15 carbon atoms and at least two hydroxyl groups. The polyols may contain other functional groups, especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols having an average molecular weight of 100 to 1,000 dalton;
- technical oligoglycerol mixtures with a degree of self-condensation of 1.5 to 10 such as, for example, technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms such as, for example, sorbitol or mannitol;
- sugars containing 5 to 12 carbon atoms such as, for example, glucose or sucrose;
- aminosugars such as, for example, glucamine;
- dialcoholamines, such as diethanolamine or 2-aminopropane-1,3-diol.

Suitable preservatives are, for example, phenoxyethanol, formaldehyde solution, parabens, pentanediol or sorbic acid. Suitable insect repellents are N,N-diethyl-m-toluamide, pentane-1,2-diol or Insect Repellent 3535. A suitable self-tanning agent is dihydroxyacetone.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. The following are preferably used either individually or in the form of mixtures: bergamot oil, dihydromyrcenol, lilial, lyral, citronellol, phenylethyl alcohol, α-hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, β-damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Suitable dyes are any of the substances suitable and approved for cosmetic purposes. These dyes are normally used in concentrations of 0.001 to 0.1% by weight, based on the mixture as a whole.

Typical examples of germ inhibitors are preservatives which act specifically against gram-positive bacteria such as, for example, 2,4,4'-trichloro-2'-hydroxydiphenyl ether, chlorhexidine (1,6-di-(4-chlorophenyl-biguanido)-hexane) or TCC (3,4,4'-trichlorocarbanilide). Numerous perfumes and essential oils also have antimicrobial properties. Typical examples are the active substances eugenol, menthol and thymol in clove, mint and thyme oil. The percentage content of the additional germ-inhibiting agents is normally about 0.1 to 2% by weight, based on the solids component of the preparations.

The total percentage content of auxiliaries and additives may be from 1 to 50% by weight and is preferably from 5 to 40% by weight, based on the particular composition. The preparations may be produced by standard hot or cold processes.

The following examples are given in order to provide a person skilled in the art with a sufficiently clear and complete explanation of the present invention, but should not be considered as limiting of the essential aspects of its subject, as set out in the preceding portions of this description.

### Examples

### Example 1. Foaming power

The foaming power of 0.1 wt.-% active matter surfactant solutions shown in table 1 below (pH 6-7; 40°C; 15°dH) was determined according to the Reverse Stirring Method^{a} in the presence of 0.5 wt.-% "Spangler sebum"^{b}.
^{a}(Castán, P.; Winkel, S.; Amela, C.; Garcia, A.; Siscart, N.; Kao Corporation SA, Spain.; "A brief study about foams. Foaming properties of amphoteric surfactants and the correlation between the determination methods applied" *Comunicaciones presentadas a las Jornadas del Comité Español* de *la Detergencia.* (1997), 27, 241-255)
^{b}[*J.Soc.Cosmet.Chem*. 32, 271-286, (July-August 1989)] (20 wt.-% olive oil + 15 wt.-% oleic acid + 15 wt.-% Coconut oil + 15 wt.-% Myristyl Myristate + 10 wt.-% palmitic acid + 10 wt.-% paraffin waxes + 5 wt.-% stearic acid + 5 wt.-% Squalane + 5 wt.-% Cholesterol)

This method consists in measuring the foam volume after agitation of 100 g of a diluted solution of the test product, placed into a 400 mL graduated cylinder, the cylinder being attached at its middle section to the axle of a motor and is rotated at 1100 r.p.m. with a metallic stirrer on a vertical plane perpendicular to this axle, changing the direction every 6 seconds during 5 minutes.

The measures were taken 5 times after 30 seconds. The values shown in table 1 are the average of the results for the mixtures according to the invention (1-2) and also for the comparative experiments (C1-C2).

**Table 1 - Foam measurements**

| Ex. | Mixture | | | | Foaming Power (mL) |
|---|---|---|---|---|---|
| | 3:1 wt.-%² | | | | |
| | | (a) | (b) | (a)/(b) wt. | |
| 1 | SLES¹ | Sodium laureth-4 carboxylate | Glycereth-7 | 6:1 | 116 |
| 2 | SLES¹ | Sodium laureth-4 carboxylate | Glycereth-9 | 6:1 | 120 |
| C1 | SLES¹ | Cocoamido propyl betaine | | | 105 |

| | | | | | |
|---|---|---|---|---|---|
| ¹Sodium laureth-2 sulfate | | | | | |
| ²SLES/surfactant or mixture of surfactants (3:1 wt.-%) | | | | | |

### Example 2. Cosmetic compositions

### 2.1. Liquid soap recipe (pH 8.5; approx. 15 wt.-% active matter)

| | |
|---|---|
| Potassium Laurate (85 % saponification) | 6 wt.-% |
| Foam Booster³ (100 % a.m.) | 5.0 wt.-% |
| PEG-4 Rapeseedamide | 3.5 wt.-% |
| Water, Perfume, Preservatives | add to 100 |

| | |
|---|---|
| ³Foam Booster: Sodium laureth-4 carboxylate and Glycereth-7 in a weight ratio of 6:1. | |

### 2.2. Other cosmetic compositions (weight percentages with respect to the composition)

| Example No | 2.2.1 | 2.2.2 | 2.2.3 | 2.2.4 | 2.2.5 |
|---|---|---|---|---|---|
| Sodium Laureth-2 Sulfate | 8 | 13 | 11 | 12 | 5 |
| Cocamidopropylbetaine | 4 | 0.5 | | 5 | 7 |
| Sodium Cocoylglutamate | | | | | 4 |
| Sodium Cocoamphoacetate | | | | 1 | |
| Decyl Glucoside | 2 | | 2 | | |
| Foam Booster³ | 0.7 | 0.5 | 3 | 1.5 | 7 |
| Glycol Distearate | | 1 | | 1 | |
| Acrylate Copolymer | 0.5 | 3 | 0.8 | | 0.5 |
| Styrene/Acrylate Copolymer | | | | 0.2 | |
| Polyquaternium-10 | 0.2 | | 0.2 | 0.2 | 0.2 |
| Hydroxypropyl Guar Hydroxypropyl Trimonium Chloride | 0.2 | 0.2 | | 0.1 | 0.1 |
| PEG-6 Caprylic/Capric Glycerides | | | | | 0.5 |
| PEG-40 Hydrogenated Castor Oil | 0.8 | 1 | | 0.2 | 0.5 |
| Glycereth-2 Cocoate | 2 | | | | |
| Paraffin Oil | | | 25 | | 0.8 |
| Soya Oil | | | 25 | | |
| Sodium benzoate | 0.45 | | 0.3 | 0.4 | |
| Sodium Salicilate | 0.2 | | 0.2 | 0.4 | |
| Citric Acid | 0.5 | 0.5 | 0.5 | 0.5 | 0.5 |
| Perfume | s.q. | s.q. | s.q. | s.q. | s.q. |
| Water | Ad 100 | Ad 100 | Ad 100 | Ad 100 | Ad 100 |

| | | | | | |
|---|---|---|---|---|---|
| ³Foam Booster: Sodium laureth-4 carboxylate and Glycereth-7 in a weight ratio of 6:1. | | | | | |

## Claims

1. Aqueous compositions comprising (a) one or more alkyl ether carboxylates of the following formula I:
R-O-(CH₂CH₂O)ₙ-CH₂COO⁻ M⁺ Formula I:
wherein
R is an alkyl residue having 6 to 22 carbon atoms;
n has a value in the range of 0.3 to 20, more preferably 0.5 to 7; still more preferably 1 to 4.5;
and M⁺ is an appropriate cation, selected from the group consisting of an alkali metal, an alkaline earth metal, ammonium, an alkylammonium, an alkanolammonium or a glucammonium;
and
(b) one or more compounds of the following formula (II): 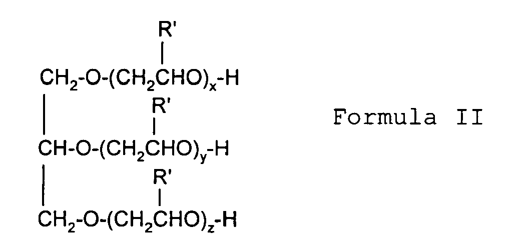
wherein
R' represents H or CH₃;
each of x, y and z independently represent a number from 0 to 25, the sum of x, y and z being in the range of 1 to 60, preferably in the range of 2 to 30, more preferably in the range of 3 to 20, even more preferably in the range of 3 to 15,
**characterized in that** the weight ratio of component (a) to component (b) is in the range of 4.1:1 to 60:1.

2. The aqueous composition according to claim 1,
**characterized in that** the weight ratio of component (a) to component (b) is in the range of 5:1 to 50:1, more preferably in the range of 6:1 to 30:1.

3. The aqueous composition according to any one of the preceding claims, additionally containing an ethoxylated fatty alcohol having the same ethoxylation degree as the alkyl ether carboxylate.

4. The aqueous composition according to claim 3, wherein the weight ratio of alkyl ether carboxylate (a) to ethoxylated fatty alcohol is in the range of 5:1 to 0,2:1, preferably 1.2:1 to 0.7:1.

5. The aqueous composition according to any one of the preceding claims, **characterized in that** in the compound of formula II R' is H.

6. The aqueous composition according to any one of the preceding claims, **characterized in that** in the compound of formula I R is an alkyl residue having 12 to 14 carbon atoms, n has a value in the range of 1 to 4.5 and M⁺ is sodium or potassium.

7. The aqueous composition according to claim 6,
**characterized in that** the compound of formula I is sodium laureth-4 carboxylate.

8. The aqueous composition according to any one of the preceding claims, **characterized in that** the compound of formula II is selected from the group consisting in Glycereth-6, Glycereth-7, Glycereth-9, Glycereth-12 or mixtures thereof.

9. The use of the aqueous composition as defined in any one of claims 1 to 8 as a foam-enhancing agent or foam booster for anionic, cationic, non-ionic and/or amphoteric surfactants or mixtures thereof.

10. Personal hygiene and/or personal care cosmetic compositions, **characterized in that** they comprise compounds as described in claims 1 to 8..

11. Cosmetic compositions according to claim 10,
**characterized in that** they are liquid soaps, hair shampoos, body shampoos, hair lotions, foam baths, shower baths, creams, gels, lotions, shaving preparations, after-shaving preparations, alcoholic and aqueous/alcoholic solutions, or emulsions.

12. Cosmetic compositions according to claims 10 or 11,
**characterized in that** the total amount of surfactants (active matter) is in the range of 10 to 35 wt.-% with respect to the total weight of the composition and the total amount of components (a) and (b) is in the range of 0.5 to 20 wt.-%, preferably 1 to 15 wt.-%, most preferred 2 to 10 wt.-%, with respect to the total weight of the composition.

13. Liquid soaps according to claims 11 to 12,
**characterized in that** they comprise:
a) an alkyl ether carboxylate of formula I as described above
b) glycerine derivatives of formula II as described above
c) one or more C12-C18 fatty acid soaps
d) water.

14. Liquid soaps according to claim 13, **characterized in that** the C12-C18 fatty acid soaps are selected from the group consisting of triethanolamine stearate, triethanolamine palmitate, triethanolamine myristate, triethanolamine laurate, sodium stearate, sodium palmitate, sodium myristate, sodium laurate, potassium stearate, potassium palmitate, potassium myristate, and potassium laurate or mixtures thereof.

15. Liquid soaps according to claims 113 or 14,
**characterized in that** it further contains (e) an ethoxylated rapeseed fatty acid amide.

16. Liquid soaps according to claims 13 to 15,
**characterized in that** they have a pH value in the range of 7 to 11.
